# EUROPEAN PATENT SPECIFICATION

(11) **EP 2 564 476 B1**
(45) Date of publication and mention of the grant of the patent: **14.01.2015**
(21) Application number: 11714766.0
(22) Date of filing: 14.04.2011
(51) Int. Cl.: H01R 24/58, H01R 43/16, H01R 43/24

(54) **MALE CONNECTOR AND A METHOD OF PRODUCING THE MALE CONNECTOR**
STECKVERBINDER UND VERFAHREN ZUR HERSTELLUNG DES STECKVERBINDERS
CONNECTEUR MÂLE ET PROCÉDÉ DE PRODUCTION DU CONNECTEUR MÂLE

(30) Priority: 30.04.2010 US 329630 P; 30.04.2010 SE 1050429
(43) Date of publication of application: 06.03.2013
(73) Proprietor: St. Jude Medical Systems AB, 751 35 Uppsala (SE)
(72) Inventor: MAHLIN, Fredrik, S-755 92 Uppsala (SE)
(74) Representative: Brann AB
(86) International application number: PCT/EP2011/055920
(87) International publication number: WO 2011/134801

(56) References cited:
- WO-A2-2009/091495
- US-A1- 2004 186 542
- US-A1- 2008 160 843
- US-A1- 2009 254 162
- US-B1- 6 439 933

## Description

### Field of the invention

The present invention relates to an injection-mouldable male connector for a medical device and a method of producing the male connector, according to the preambles of the independent claims.

### Background of the invention

In many medical procedures, various physiological conditions present within a body cavity need to be monitored. These physiological conditions are typically physical in nature - such as pressure, temperature, rate-of-fluid flow, and provide the physician or medical technician with critical information as to the status of a patient's condition.

For example, one medical device that is widely used to monitor conditions is a intravascular pressure sensor. The pressure sensor senses the magnitude of a patient's blood pressure, and converts it into a representative electrical signal that is transmitted to the exterior of the patient. For most applications it is also required that the sensor is electrically energized. Some means of signal and energy transmission for such a medical device is thus required.

For the blood pressure sensor, most commonly, extremely thin electrical cables, sometimes called microcables, are provided inside a guide wire, which itself preferably is provided in the form of a tube, which often has an outer diameter in the order of 0.35 mm, and oftentimes is made of steel. In order to increase the bending strength of the tubular guide wire and improve pushability and torque response, a core wire may be positioned inside the tube. The mentioned electrical cables are then e.g. positioned in the space between the inner lumen wall and the core wire.

In a guide wire mounted sensor, the signals from the sensor, arranged at the distal end of the guide wire, are lead through the electrical leads to a male connector at the proximal end of the guide wire. In use, the male connector is connected to a corresponding female connector and the signals from the pressure sensor are transferred to an interface, which converts the signals and presents them in a desired form for an operator.

A conventional male connector for a medical device, basically comprises a core wire, a plurality of conductors, a plurality of conductive members, and insulating material therebetween. When the male connector is connected to the female connector, the conductive members transfer the signals from the conductors of the male connector to similar conductive members inside the female connector.

Several different designs of male connectors are known in the prior art, and examples of such male connectors are disclosed in US 6196980 B1, US 6090052 A, and US 6908442 B2, which are assigned to the same assignee as in the present application.

A male connector according to the preamble of the independent claim 1 is known from WO-A-2009091495.

For example, the male connector used today for guide wires is made by individually attaching each conductor (an electrical lead placed alongside a core wire) to a conductive member (a contact ring which is wrapped around the core wire). Each of the conductive members are connected, by bonding or soldering, respectively, to a conductor. The core wire is used to prevent kinks and to provide strength to the male connector. Especially when the male connector is inserted into the female connector, there is a substantial risk of over-bending the male connector or damaging the thin conductors inside the connector.

Soldering or bonding the conductive members to the conductors is a time-consuming process and may be a source of manufacturing mistakes, leading to loss of time and material in the manufacturing process. For example, there may be a risk that the conductive members and the conductors are not properly connected or that they disconnect due to over-bending of the male connector and this in its turn involves a risk for short circuit in the male connector.

Consequently, there remains a need for a male connector which is less expensive, easy and straightforward to manufacture and which reduces the risk of short circuit. In addition, there is a need for a male connector which is possible to test before assembling with the medical device.

There is also a need for a male connector which makes it possible to bond or solder all the electrical cables at one end of the finished module, whereby the male connector may be mounted in one piece to electrical cable(s) for e.g. a sensor guide wire.

### Summary of the invention

The above-mentioned objects are achieved by the present invention according to the independent claim 1.

Preferred embodiments are set forth in the dependent claims.

Thus, the object of the present invention is to achieve an improved male connector that is easier to manufacture and assemble than presently used male connectors, and which mechanically is more stable and which not tend to kink when inserted into a female connector.

Further, the object of the present invention is to provide a method of manufacturing a male connector by injection-moulding; according to claim 10.

The elongated male connector, for a medical device, in accordance with the present invention, has the features of the independent claim 1.

The invention will now be described in detail with reference to the drawings.

### Short description of the appended drawings

Figure 1 shows the male connector according to the present invention.
Figure 2 shows a close-up view of the male connector according a first preferred embodiment of the present invention.
Figure 3 shows a single conductive member provided with a microrod.
Figure 4 shows the male connector, according to a second preferred embodiment of the present invention, in which figure, the insulating members have been omitted.
Figure 5 shows the distal end of the male connector, according to a second embodiment of the present invention, in which figure, the insulating members have been omitted.

Throughout the figures the same reference signs designate the same, or essentially the same features.

### Detailed description of preferred embodiments of the invention

Throughout the application the word distal refers to the part located furthest away in respect of the operator, and the word proximal refers to the part located closest in respect of the operator.

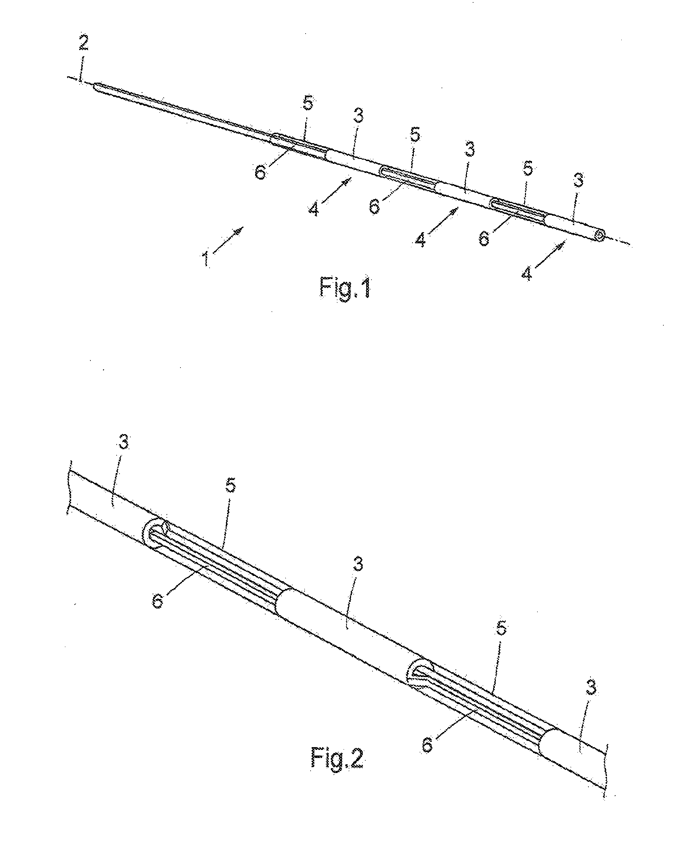

In Figure 1, an elongated male connector 1, for a medical device, according to the present invention, is disclosed. The male connector 1 has a longitudinal axis 2 and comprises a plurality of conductive members 3, each having an outer contact surface 4 and being separated from each other by insulating members 5, the conductive and insulating members 3, 5 being disposed along the male connector 1, such that the outer contact surfaces 4 are arranged essentially at the same surface level, and that each of the conductive members 3 has an elongated extension along the longitudinal axis 2 of the male connector 1.

As illustrated in figure 2, each conductive member 3 is provided with an insulated microrod 6 extending at least partially along the length of the male connector 1. The conductive members 3 are hollow and have an essentially cylindrical cross-section and form, together with the insulating members 5, a self-supporting male connector 1 having no core wire. Thus, the male connector 1 consists merely of the conductive and insulating members 3, 5 and the insulated microrods 6.

The male connector is adapted to any medical device adapted to be inserted into the body. The medical device may be e.g a sensor guide wire for intravascular measurements of physiological variables in a living body. The sensor may be designed to measure temperature and/or flow e.g. within the vascular system. Miniature sensors may also be designed to measure other variables, such as magnetic flux, other electromagnetic variables, conductance, or inductance, e.g. generated from outside the body and used inter alia for positioning purposes.

As discussed above, and as shown in figures 1-2, the outer contact surfaces 4 are arranged essentially at the same surface level. However, as an obvious constructive variation, the surface level of the conductive members 3 may be arranged slightly elevated in respect to the surface level of the insulating members 5, i.e. the radius of the conductive members 3 is larger than the radius of the insulating members 5. As an example, the conductive members have a cross-sectional diameter of 0,2 - 0,4 mm.

According to a preferred embodiment of the present invention, the microrods 6 are arranged to extend through more distally arranged conductive and insulating members 3, 5, as illustrated in figures 1 and 2. The insulating members 5 are preferably made of LCP (Liquid Crystal Polymer), and the LCP is preferably injection-moulded onto the assembled male connector 1. However, other suitable materials, such as PEI (polyetherimide) or PEEK (polyetheretherketone), may also be used to insulate between the conductive members 3.

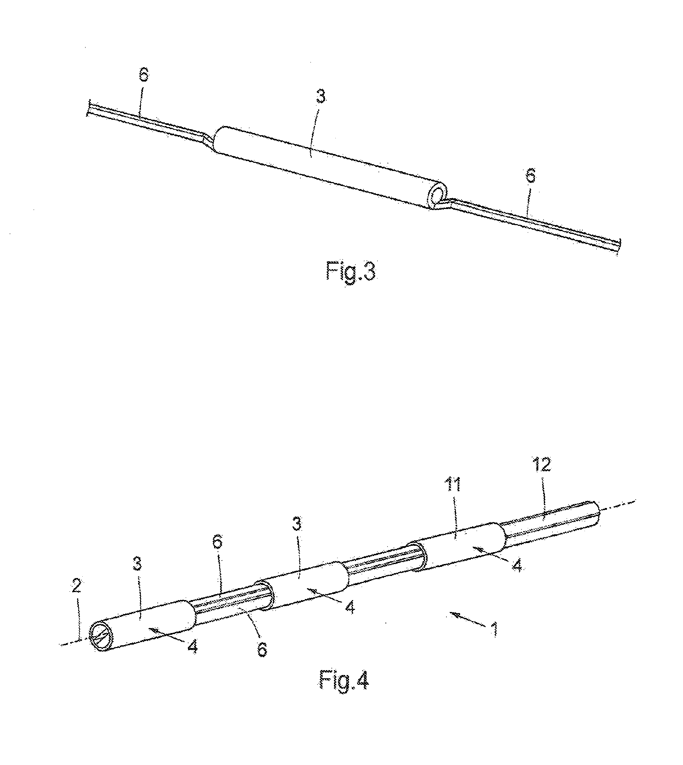

Figure 3, shows a single conductive member 3, of a male connector 1 according to a first preferred embodiment of the present invention. In this preferred embodiment, the conductive members 3 have a circular cross-section, and the microrods 6 are adapted to be arranged to extend through more distally and more proximally arranged conductive and insulating members 3, 5. The microrods 6, connected to the conductive members 3, are preferably insulated with polyimide, or other suitable insulating material, such as oxide coating or acrylic insulation. The conductive members 3 provided with the microrods 6 are according to this preferred embodiment manufactured by cutting out separate units of conductive members 3 provided with microrods 6, preferably by laser, from an elongated tube (not shown).

In another embodiment, the conductive members 3 provided with microrods 6 may be produced by laser cutting a flat sheet of conductive material, e.g. sheet metal, and bent or rolled into a cylindrical shape.

According to another preferred embodiment of the present invention, shown in figure 4, in which figure the insulating members have been omitted, the microrods 6 are arranged to extend through more distally, or both through more distally and more proximally, arranged conductive and insulating members 3, 5. Since the male connector 1 has no core wire, the microrods 6 instead support the conductive and insulating members 3, 5, to achieve a mechanically stable male connector 1 which does not kink. Thus, the microrods 6 support the conductive and insulating members 3, 5 and give the male connector 1 the necessary stability and stiffness.

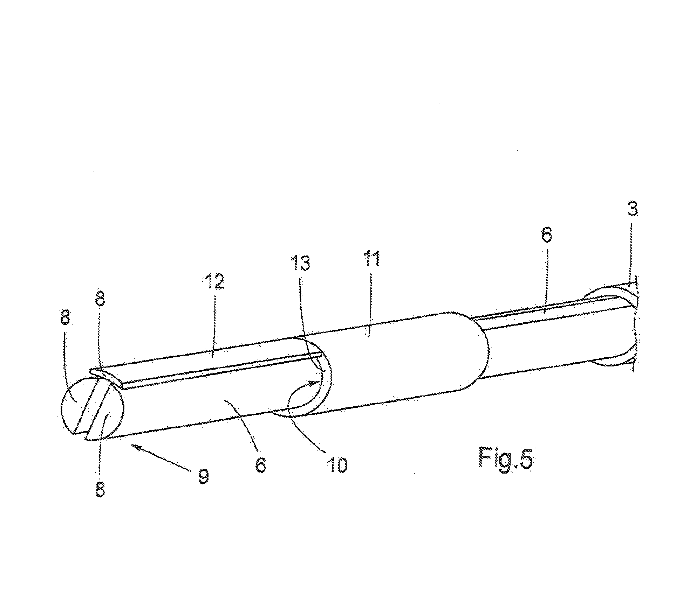

In other embodiments the microrods 6 instead are produced separately, and thereafter fastened to the conductive members 3. This may be achieved e.g. by means of spot-welding, or soldering, according to the embodiment shown in figures 4 and 5. However, other suitable techniques, such as welding or gluing, may also be used to attach the microrods 6 to the conductive members 3. At least one of the microrods 6 are, according to this embodiment, fastened to the inner space 10 of the hollow conductive members 3.

According to one embodiment of the present invention, the microrods 6 are provided with contact surfaces 8 at a distal end 9 of the male connector 1, as shown in detail in figure 5. When the male connector 1 is in use, the contact surfaces 8 are connected, preferably by welding, to electrical leads provided in the medical device, e.g. a sensor guide wire. Contact surfaces 8 at one end of the male connector 1 is advantageous since it makes it possible to test the male connector 1 before assembling with the medical device. Thereby, the risk for short circuit in the male connector module 1 is reduced. Also, having all the contact surfaces adjacent to each other at one end simplifies connecting several electrical leads to the male connector.

In the embodiment illustrated in figure 5, the microrods 6, have a semilunar cross-section. The dimensions of the semilunar microrods 6 are adapted to the diameter of the inner space 10 of the hollow conductive members 3, so that one or more microrods 6 may be arranged to extend through the conductive member 3 supported by the inner space 10 of the conductive members 3 to make the male connector 1 mechanically stable. As shown in figures 4 and 5, the most distal arranged conductive member 11 may be provided with a distal microrod 12 having less dimensions than the other microrods 6 attached to the more proximal arranged conductive members 3. The distal microrod 12 is preferably attached to the distal edge 13 of the most distal arranged conductive member 11.

The present invention also relates to a method of producing the male connector 1, for a medical device. The method includes:
a) providing a plurality of hollow elongated conductive members 3 having a cylindrical cross-section and an outer contact surface 4, where each conductive member 3 is provided with an elongated microrod 6; b) insulating each conductive member and microrod with a suitable
b) insulating each conductive member and microrod with a suitable insulating material, such as polyimide, oxide coating or acrylic insulation;
c) arranging, spaced apart, a plurality of the conductive members 3 next to each other and centred along a longitudinal axis 2 with the microrods 6 extending through more distally arranged conductive members 3, and
d) providing insulating members 5 between adjacent conductive members 3, thereby forming a self-supporting male connector 1 having no core wire.

According to the method for producing a male connector 1, step b) may further include the sub step of:
b1) arranging at least one of the microrods 6 to extend through at least one proximally arranged conductive member 3.

As a preferred method, and according to the preferred embodiment of the present invention shown in figures 1-3, the hollow elongated conductive members 3 provided with the microrods 6, are manufactured at the same time, by cutting out separate units, preferably by laser, from an elongated tube, made of any commonly used conductive material, such as stainless steel, platinum or titanium. Each cut out conductive member 3 and microrod 6 are then insulated with a suitable insulating material, such as polyimide, oxide coating or acrylic insulation as is common in the art.

As also discussed above, and according to another embodiment, the conductive members 3 provided with microrods 6 may be produced by laser cutting a flat sheet of conductive material, e.g. sheet metal, and bent or rolled into a cylindrical shape.

However, in the embodiment illustrated in figures 4-5, as discussed above, the microrods 6 are fastened to the conductive members 3 by means of spot-welding.

According to the method, the insulating members 5 may be provided by means of injection-moulding. In detail, the conductive members 3 provided with the microrods 6 are then arranged spaced apart, and next to each other, and adjacent conductive member 3 are threaded on to adjacent microrods 6, so that the microrods 6 extends through more distally and/or proximally arranged conductive members 3, as shown in figures 4-5. Subsequently, the insulation is injection-moulded onto the microrods 6, between adjacent conductive members 3, in order to form the insulating members 5.

Furthermore, and according to the method, the microrods 6 are bonded or soldered at a distal end 9 of the male connector 1 to form a plurality of contact surfaces 8. Advantageously, all cable connector contact surfaces 8 to be connected to the desired medical device are then arranged at one end of the male connector 1.

The present invention is not limited to the above-described preferred embodiments. Various alternatives and modifications may be used. Therefore, the above embodiments should not be taken as limiting the scope of the invention, which is defined by the appending claims.

## Claims

1. An elongated male connector (1) for a medical device, the male connector (1) having a longitudinal axis (2) and comprises:
- a plurality of conductive members (3), each having an outer contact surface (4) and being separated from each other by insulating members (5), the conductive and insulating members (3, 5) being disposed along the male connector (1), such that the outer contact surfaces (4) are arranged essentially at the same surface level, and that each of the conductive members (3) has an elongated extension along the longitudinal axis (2) of the male connector (1),
**characterized in that** each conductive member (3) is provided with a microrod (6) extending at least partially along the length of the male connector (1), wherein said microrods (6) are provided with contact surfaces (8) at a distal end (9) of said male connector (1), and said microrods (6) are provided with an insulating coating such that electrical current can flow between each conductive member (3) via a microrod (6) to the contact surface (8), and that said conductive members (3) are hollow and have an essentially cylindrical cross-section and form, together with said insulating members (5), a self-supporting male connector (1) having no core wire, wherein the microrods (6) support the conductive members (3) and the insulating members (5) to achieve a mechanically stable male connector.

2. An elongated male connector (1) according to claim 1, wherein said microrods (6) are arranged to extend through more distally arranged conductive and insulating members (3, 5).

3. An elongated male connector (1) according to any of claims 1-2, wherein said conductive members (3) have a circular cross-section.

4. An elongated male connector (1) according to any of claims 1-3, wherein said conductive members (3) are made of a flat sheet of conductive material, e.g. a sheet metal, which is bent or rolled into a cylindrical shape.

5. An elongated male connector (1) according to any of claims 1-4, wherein at least one of said microrods (6) is arranged to extend through more proximally arranged conductive and insulating members (3, 5).

6. An elongated male connector (1) according to any of claims 1-5, wherein said insulating members (5) are made of LCP (Liquid Crystal Polymer).

7. An elongated male connector (1) according to any of claims 1-6, wherein said microrods (6) are fastened to said conductive members (3) by means of spot-welding.

8. An elongated male connector (1) according to any of claims 1-6, wherein said microrods (6) are fastened to said conductive members (3) by means of soldering.

9. An elongated male connector (1) according to any of claims 1-8, wherein said microrods (6) have a semilunar cross-section.

10. Method of producing a male connector (1) according to claim 1, for a medical device, the method includes:
a) providing a plurality of hollow elongated conductive members (3) having a cylindrical cross-section and an outer contact surface (4), where each conductive member (3) is provided with an elongated microrod (6);
b) insulating each conductive member (3) and microrod (6) with a suitable insulating material, such as polyimide, oxide coating or acrylic insulation;
c) arranging, spaced apart, a plurality of said conductive members (3) next to each other and centred along a longitudinal axis (2) with said microrods (6) extending through more distally arranged conductive members (3), and
d) providing insulating members (5) between adjacent conductive members (3) thereby forming a self-supporting male connector (1) having no core wire.

11. Method for producing a male connector (1) according to claim 10, where step b) further includes the sub step of:
b1) arranging at least one of said microrods (6) to extend through at least one proximally arranged conductive member (3).

12. Method according to any of claims 10-11, wherein said insulating members (5) are provided by means of injection-moulding.

13. Method according to any of claims 10-12, wherein said microrods (6) are fastened to said conductive members (3) by means of spot-welding.

14. Method according to any of claims 10-12, wherein said microrods (6) are fastened to said conductive members (3) by means of soldering.

15. Method according to any of claims 10-12, wherein said conductive members (3) provided with said microrods (6) are provided by cutting out separate units of conductive members (3) provided with microrods (6), preferably by laser, from an elongated tube.

16. Method according to any of claims 10-12, wherein said conductive members (3) are provided by laser cutting a flat sheet of conductive material, e.g. a sheet metal, which subsequently is bent or rolled into a cylindrical shape.

17. Method according to any of claims 10-16, wherein said microrods (6) are bonded or soldered at a distal end (9) of said male connector (1) to form a plurality of contact surfaces (8).

## Patentansprüche

1. Länglicher Steckverbinder (1) für eine medizinische Vorrichtung, wobei der Steckverbinder (1) eine Längsachse (2) hat und Folgendes umfasst:
- mehrere leitfähige Elemente (3), von denen jedes eine äußere Kontaktfläche (4) hat und die voneinander durch isolierende Elemente (5) getrennt sind, wobei die leitfähigen und isolierenden Elemente (3, 5) entlang des Steckverbinders (1) derart angeordnet sind, dass die äußeren Kontaktflächen (4) im Wesentlichen auf demselben Flächenniveau angeordnet sind und dass jedes der leitfähigen Elemente (3) eine gestreckte Verlängerung entlang der Längsachse (2) des Steckverbinders (1) hat,
**dadurch gekennzeichnet, dass** jedes leitfähige Element (3) mit einem Mikrostab (6) versehen ist, der sich zumindest teilweise entlang der Länge des Steckverbinders (1) erstreckt, wobei die Mikrostäbe (6) mit Kontaktflächen (8) an einem distalen Ende (9) des Steckverbinders (1) versehen sind und die Mikrostäbe (6) mit einer isolierenden Beschichtung derart versehen sind, dass elektrischer Strom zwischen jedem leitfähigen Element (3) über einen Mikrostab (6) zur Kontaktfläche (8) fließen kann und dass die leitfähigen Elemente (3) hohl sind und einen im Wesentlichen zylindrischen Querschnitt haben und zusammen mit den isolierenden Elementen (5) einen selbsttragenden Steckverbinder (1) bilden, der keinen Kerndraht hat, wobei die Mikrostäbe (6) die leitfähigen Elemente (3) und die isolierenden Elemente (5) stützen, um einen mechanisch stabilen Steckverbinder zu erzielen.

2. Länglicher Steckverbinder (1) nach Anspruch 1, wobei die Mikrostäbe (6) zum Erstrecken durch mehr distal angeordnete leitfähige und isolierende Elemente (3, 5) ausgelegt sind.

3. Länglicher Steckverbinder (1) nach einem der Ansprüche 1-2, wobei die leitfähigen Elemente (3) einen kreisförmigen Querschnitt haben.

4. Länglicher Steckverbinder (1) nach einem der Ansprüche 1-3, wobei die leitfähigen Elemente (3) aus einem flachen Blatt von leitfähigem Material hergestellt sind, z.B. einem Blech, das in eine zylindrische Form gebogen oder gerollt ist.

5. Länglicher Steckverbinder (1) nach einem der Ansprüche 1-4, wobei mindestens einer der Mikrostäbe (6) zum Erstrecken durch mehr proximal angeordnete leitfähige und isolierende Elemente (3, 5) ausgelegt ist.

6. Länglicher Steckverbinder (1) nach einem der Ansprüche 1-5, wobei die isolierenden Elemente (5) aus LCP (Flüssigkristallpolymer) hergestellt sind.

7. Länglicher Steckverbinder (1) nach einem der Ansprüche 1-6, wobei die Mikrostäbe (6) an den leitfähigen Elementen (3) mittels Punktschweißen befestigt sind.

8. Länglicher Steckverbinder (1) nach einem der Ansprüche 1-6, wobei die Mikrostäbe (6) an den leitfähigen Elementen (3) mittels Löten befestigt sind.

9. Länglicher Steckverbinder (1) nach einem der Ansprüche 1-8, wobei die Mikrostäbe (6) einen halbmondförmigen Querschnitt haben.

10. Verfahren zum Erzeugen eines Steckverbinders (1) nach Anspruch 1 für eine medizinische Vorrichtung, wobei das Verfahren Folgendes umfasst:
a) Bereitstellen mehrerer hohler länglicher leitfähiger Elemente (3), die einen zylindrischen Querschnitt und eine äußere Kontaktfläche (4) haben, wobei jedes leitfähige Element (3) mit einem länglichen Mikrostab (6) versehen ist;
b) Isolieren jedes leitfähigen Elementes (3) und Mikrostabs (6) mit einem geeigneten Isoliermaterial, wie zum Beispiel Polyimid, Oxidbeschichtung oder Acrylisolation;
c) Anordnen mehrerer der leitfähigen Elemente (3) mit Abstand nebeneinander und zentriert entlang einer Längsachse (2), wobei sich die Mikrostäbe (6) durch mehr distal angeordnete leitfähige Elemente (3) erstrecken, und
d) Bereitstellen von isolierenden Elementen (5) zwischen benachbarten leitfähigen Elementen (3), dadurch Bilden eines selbsttragenden Steckverbinders (1), der keinen Kerndraht hat.

11. Verfahren zum Herstellen eines Steckverbinders (1) nach Anspruch 10, wobei Schritt b) ferner den folgenden Teilschritt umfasst:
b1) Anordnen von mindestens einem der Mikrostäbe (6), so
dass er sich durch mindestens ein proximal angeordnetes leitfähiges Element (3) erstreckt.

12. Verfahren nach einem der Ansprüche 10-11, wobei die isolierenden Elemente (5) mittels Spritzgießen bereitgestellt werden.

13. Verfahren nach einem der Ansprüche 10-12, wobei die Mikrostäbe (6) an den leitfähigen Elementen (3) mittels Punktschweißen befestigt sind.

14. Verfahren nach einem der Ansprüche 10-12, wobei die Mikrostäbe (6) an den leitfähigen Elementen (3) mittels Löten befestigt sind.

15. Verfahren nach einem der Ansprüche 10-12, wobei die leitfähigen Elemente (3), die mit den Mikrostäben (6) versehen sind, durch Ausschneiden separater Einheiten von leitfähigen Elementen (3), die mit Mikrostäben (6) versehen sind, aus einem länglichen Rohr, vorzugsweise durch Laser, bereitgestellt werden.

16. Verfahren nach einem der Ansprüche 10-12, wobei die leitfähigen Elemente (3) durch Laserschneiden eines flachen Blattes von leitfähigem Material bereitgestellt werden, z.B. eines Bleches, das anschließend zu einer zylindrischen Form gebogen oder gerollt wird.

17. Verfahren nach einem der Ansprüche 10-16, wobei die Mikrostäbe (6) an einem distalen Ende (9) des Steckverbinders (1) zusammengefügt oder gelötet werden, um mehrere Kontaktflächen (8) zu bilden.

## Revendications

1. Connecteur mâle allongé (1) pour un dispositif médical, le connecteur mâle (1) ayant un axe longitudinal (2) et comprenant :
une pluralité d'organes conducteurs (3), ayant chacun une surface de contact externe (4) et étant séparés les uns des autres par des organes isolants (5), les organes conducteurs et isolants (3, 5) étant disposés le long du connecteur mâle (1), de sorte que les surfaces de contact externes (4) soient agencées essentiellement au même niveau de surface, et de sorte que chacun des organes conducteurs (3) ait un prolongement allongé le long de l'axe longitudinal (2) du connecteur mâle (1),
**caractérisé en ce que** chaque organe conducteur (3) est pourvu d'une microtige (6) s'étendant au moins partiellement selon la longueur du connecteur mâle (1), dans lequel lesdites microtiges (6) sont pourvues de surfaces de contact (8) à une extrémité distale (9) dudit connecteur mâle (1), et lesdites microtiges (6) sont pourvues d'un revêtement isolant de sorte qu'un courant électrique puisse circuler entre chaque organe conducteur (3) via une microtige (6) vers la surface de contact (8), et **en ce que** lesdits organes conducteurs (3) sont creux et ont une section essentiellement cylindrique et forment, conjointement avec lesdits organes isolants (5), un connecteur mâle autoportant (1) dépourvu de fil de coeur, dans lequel les microtiges (6) supportent les organes conducteurs (3) et les organes isolants (5) pour réaliser un connecteur mâle stable mécaniquement.

2. Connecteur mâle allongé (1) selon la revendication 1, dans lequel lesdites microtiges (6) sont agencées pour s'étendre à travers des organes conducteurs et isolants (3, 5) agencés de façon plus distale.

3. Connecteur mâle allongé (1) selon l'une quelconque des revendications 1 à 2, dans lequel lesdits organes conducteurs (3) ont une section circulaire.

4. Connecteur mâle allongé (1) selon l'une quelconque des revendications 1 à 3, dans lequel lesdits organes conducteurs (3) sont constitués d'une feuille plate de matériau conducteur, par exemple une feuille en métal, qui est cintrée ou laminée en une forme circulaire.

5. Connecteur mâle allongé (1) selon l'une quelconque des revendications 1 à 4, dans lequel au moins l'une desdites microtiges (6) est agencée pour s'étendre à travers des organes conducteurs et isolants (3, 5) agencés de façon plus proximale.

6. Connecteur mâle allongé (1) selon l'une quelconque des revendications 1 à 5, dans lequel lesdits organes isolants (5) sont constitués de PCL (polymère à cristaux liquides).

7. Connecteur mâle allongé (1) selon l'une quelconque des revendications 1 à 6, dans lequel lesdites microtiges (6) sont fixées auxdits organes conducteurs (3) au moyen d'un soudage par points.

8. Connecteur mâle allongé (1) selon l'une quelconque des revendications 1 à 6, dans lequel lesdites microtiges (6) sont fixées auxdits organes conducteurs (3) au moyen d'un brasage.

9. Connecteur mâle allongé (1) selon l'une quelconque des revendications 1 à 8, dans lequel lesdites microtiges (6) ont une section semi-lunaire.

10. Procédé de production d'un connecteur mâle (1) selon la revendication 1, pour un dispositif médical, le procédé comprenant :
a) la fourniture d'une pluralité d'organes conducteurs allongés creux (3) ayant une section cylindrique et une surface de contact externe (4), où chaque organe conducteur (3) est pourvu d'une microtige (6) allongée ;
b) l'isolation de chaque organe conducteur (3) et de la microtige (6) avec un matériau isolant approprié, tel que le poly(imide), un revêtement en oxyde ou une isolation acrylique ;
c) l'agencement, de façon espacée, d'une pluralité desdits organes conducteurs (3) près les uns des autres et centrés le long d'un axe longitudinal (2) avec lesdites microtiges (6) s'étendant à travers des organes conducteurs (3) agencés de façon plus distale, et
d) la fourniture d'organes isolants (5) entre les organes conducteurs (3) adjacents, formant ainsi un connecteur mâle autoportant (1) dépourvu de fil de coeur.

11. Procédé de production d'un connecteur mâle (1) selon la revendication 10, où l'étape b) inclut en outre la sous-étape de :
b1) agencement d'au moins l'une desdites microtiges (6) pour qu'elle s'étende à travers au moins un organe conducteur (3) agencé de façon proximale.

12. Procédé selon l'une quelconque des revendications 10 à 11, dans lequel lesdits organes isolants (5) sont fournis au moyen d'un moulage par injection.

13. Procédé selon l'une quelconque des revendications 10 à 12, dans lequel lesdites microtiges (6) sont fixées auxdits organes conducteurs (3) au moyen d'un soudage par points.

14. Procédé selon l'une quelconque des revendications 10 à 12, dans lequel lesdites microtiges (6) sont fixées auxdits organes conducteurs (3) au moyen d'un brasage.

15. Procédé selon l'une quelconque des revendications 10 à 12, dans lequel lesdits organes conducteurs (3) pourvus desdites microtiges (6) sont fournis en découpant des unités séparées d'organes conducteurs (3) pourvus de microtiges (6), de préférence par laser, à partir d'un tube allongé.

16. Procédé selon l'une quelconque des revendications 10 à 12, dans lequel lesdits organes conducteurs (3) sont fournis par découpe laser d'une feuille plate de matériau conducteur, par exemple une feuille de métal, qui est ultérieurement cintrée ou laminée en une forme cylindrique.

17. Procédé selon l'une quelconque des revendications 10 à 16, dans lequel lesdites microtiges (6) sont liées ou brasées à une extrémité distale (9) dudit connecteur mâle (1) pour former une pluralité de surfaces de contact (8).
